# EUROPÄISCHE PATENTSCHRIFT

(11) **EP 0 979 809 B1**
(45) Veröffentlichungstag und Bekanntmachung des Hinweises auf die Patenterteilung: **29.01.2003**
(21) Anmeldenummer: 99115366.9
(22) Anmeldetag: 04.08.1999
(51) Int. Cl.: C07C 17/26, C07C 25/18, C07C 41/30, C07C 43/225, C07B 49/00, B01J 31/22, B01J 31/24

(54) **Vefahren zur Herstellung von 4-Chlorbiphenylen**
Process for the preparation of 4-chlorobiphenylenes
Procédé pour la préparation de 4-chlorobiphénylènes

(30) Priorität: 12.08.1998 DE 19836470
(43) Veröffentlichungstag der Anmeldung: 16.02.2000
(73) Patentinhaber: Clariant GmbH, 65929 Frankfurt am Main (DE)
(72) Erfinder: Haber, Steffen, Dr., 61462 Königstein (DE); Scherer, Stefan, Dr., 64572 Büttelborn (DE); Meudt, Andreas, Dr., 65439 Flörsheim-Weilbach (DE); Nörenberg, Antje, Dr., 64572 Büttelborn (DE)

(56) Entgegenhaltungen:
- DATABASE WPI Section Ch, Week 9304 Derwent Publications Ltd., London, GB; Class B05, AN 93-030491 XP002116333 & JP 04 356431 A (HOKKO CHEM IND CO LTD), 10. Dezember 1992 (1992-12-10)

## Beschreibung

Die vorliegende Erfindung betrifft ein neues Verfahren zur Herstellung von 4-Chlorbiphenylen aus Halogenaromaten und Arylgrignardreagenzien mit einem Phosphino-palladium-ferrocen-Katalysator. 4-Chlorbiphenyle haben technische Bedeutung als Wirkstoffvorprodukte für Matrix-Metalloprotease-Inhibitoren.

Eine häufig angewandte Methode zur Synthese von 4-Chlorbiphenylen im Labormaßstab sind palladiumkatalysierte Kreuzkupplungen, bei der lod- und Bromaromaten mit metallorganischen Arylderivaten, insbesondere Arylboronsäuren oder Arylgrignardreagenzien in Gegenwart von Palladium- oder Nickelkatalysatoren umgesetzt werden. Beispiele, die diese Methodik beschreiben, findet man beispielsweise in Y. Ikoma et al., Synthetic Commun. 21 (1991), 3, 481-487.

Trotz der Vielzahl von Veröffentlichungen auf dem Gebiet der Synthese von 4-Chlorbiphenylen in Gegenwart von Nickel- bzw. Palladiumkatalysatoren sind bisher keine Beispiele für eine größere technische Umsetzung der Methoden bekannt. Dies ist darauf zurückzuführen, daß die beschriebenen Katalysatorsysteme häufig nur mit nicht ökonomischen Katalysatormengen oder geringen Selektivitäten, d.h. hohen Raten an Dimerisierungsprodukten und dechlorierten Produkten, einsetzbar sind (A. Hutz et al., Tetrahedron, 45, 21, 1989, 6679-6682). Ansonsten müssen große Mengen an Katalysator - allgemein > 1 mol % - zugesetzt werden, um technisch nutzbare Umsätze zu erzielen. Aufgrund der Komplexität der Reaktionsgemische ist zudem kein einfaches Katalysatorrecycling möglich, so daß Katalysatorkosten in der Regel eine technische Realisierung verhindern.

Außerdem beobachtet man bei der Suzuki-Kupplung substituierter Biphenyle mit gängigen Katalysatorsystemen, wie Pd(OAc)₂/Triphenylphosphan-Gemischen, als Nebenreaktion Arylübertragungen (O'Keefe et al., Tetrahedron Letters 1992, 6679).

In JP 04 356 431 wird die Herstellung von 4-Chlorbiphenylen ausgehend von Dichlorobenzol, einem Grignard-Reagenz und einem Pd Katalysator beschrieben.

Aus den genannten Gründen ist es von großem industriellem Interesse, bessere, technisch nutzbare Katalysatorsysteme für die Synthese von 4-Chlorbiphenylen, insbesondere für die Arylierung von ökonomisch günstigen Brom- und Chloraromaten zu finden. Es bestand somit ein großer Bedarf nach einem Verfahren, das die beschriebenen Nachteile vermeidet und 4-Chlorbiphenyle in hoher Reinheit in technisch einfacher Weise zugänglich macht.
Diese Aufgabe wird gelöst durch ein Verfahren zur Herstellung von 4-Chlorbiphenylen der Formel (I) 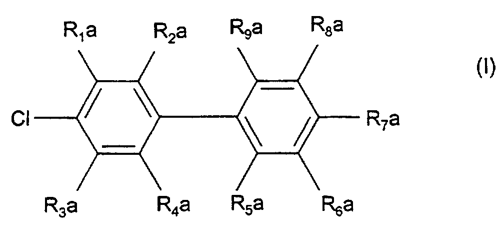 worin
R₁a bis R₄a gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, C₁-C₁₂-Acyloxy, O-Phenyl, O-Benzyl, Aryl, Heteroaryl, F, Cl, NO₂, CN, SO₂R, SOR, NH(C₁-C₆-alkyl), N(C₁-C₆alkyl)₂, C=N(C₁-C₆-alkyl), CX₃, COO-(C₁-C₁₂-alkyl), CO-(C₁-C₁₂-alkyl), CO-Phenyl, COO-Phenyl, CON(C₁-C₈-alkyl)₂, CONH(C₁-C₈-alkyl), CHCHCOO-(C₁-C₁₂-alkyl), N(Si(C₁-C₄-alkyl)₃), PO(Phenyl)₂, PO-(C₁-C₈-alkyl)₂ oder PO₃-(C₁-C₈-alkyl)₂ haben, wobei R Aryl, vorzugsweise Phenyl oder Naphthyl, F oder CₙF₂ₙ₊₁ mit n = 1 bis 12 ist, und wobei X F, Cl oder Br ist; und
R₅a bis R₉a gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, O-Phenyl, O-Benzyl, Aryl, Heteroaryl, F, Cl, NO₂, CN, SO₂R, SOR, wobei R Aryl, vorzugsweise Phenyl oder Naphthyl ist, haben,
dadurch gekennzeichnet, daß ein Halogenaromat der Formel (II) 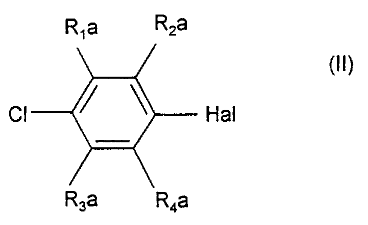 mit einer Arylgrignardverbindung der Formel (III) 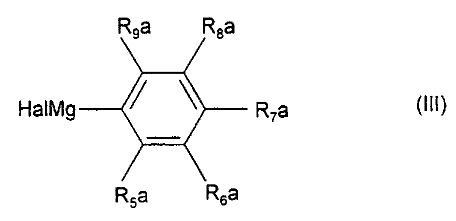 worin Hal für Brom oder Jod steht, in Gegenwart eines Palladiumkatalysators der Formel (IV) 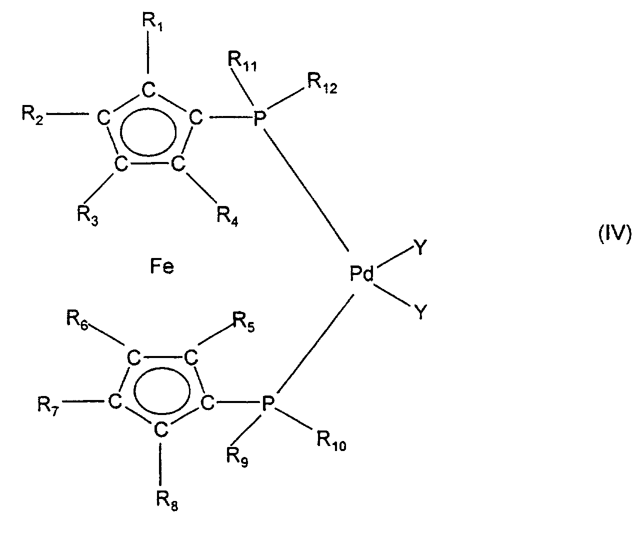 worin
R₁ bis R₈ gleich oder verschieden sind und Wasserstoff, (C₁-C₄)-Alkyl, (C₅-C₈)-Cycloalkyl, (C₁-C₄)-Alkoxy, Fluor, NH₂, NH-(C₁-C₄-Alkyl), N(C₁-C₄-alkyl)₂, CO₂-Alkyl-(C₁-C₄) oder Phenyl bedeuten, oder R₁ und R₂, oder R₂ und R₃, oder R₃ und R₄; und/oder R₅ und R₆, oder R₆ und R₇, oder R₇ und R₈ zusammen einen aliphatischen oder aromatischen Ring bilden,
R₉ bis R₁₂ gleich oder verschieden sind und C₁-C₈-Alkyl, C₃-C₁₂-Cycloalkyl oder Aryl, das durch 1 bis 3 Substituenten aus der Gruppe C₁-C₄-Alkyl, C₁-C₄-Alkoxy und Halogen substituiert sein kann, bedeuten, und
Y ein Anion einer organischen oder anorganischen Säure ist, umgesetzt wird.

Bevorzugt ist ein Verfahren zur Herstellung von Verbindungen der Formel (I), worin R₁a bis R₄a Wasserstoff, C₁-C₈-Alkyl, C₁-C₈-Alkoxy, F, Cl, CN, ein 5- oder 6-gliedriges Heteroaryl mit 1 bis 3 Heteroatomen aus der Gruppe O, S und N, COO-(C₁-C₈-alkyl), CONH(C₁-C₄-alkyl) oder CON(C₁-C₄-alkyl)₂ bedeuten und worin R₅a bis R₉a Wasserstoff, C₁-C₆-Alkyl, C₂-C₆-Alkenyl, C₁-C₆-Alkoxy, O-Phenyl, O-Benzyl, F, Cl, NH(C₁-C₄-alkyl) oder N(C₁-C₄-alkyl)₂ bedeuten.

Besonders bevorzugt ist ein Verfahren zur Herstellung von Verbindungen der Formel (I), worin
R₁a bis R₄a Wasserstoff, C₁-C₄-Alkyl, C₁-C₄-Alkoxy, CN, COO(C₁-C₄-alkyl), Phenyl, Thiophenyl, Furanyl, Imidazolyl, Oxadiazolyl, Thiadiazolyl, Oxazolyl,
4,5-Dihydrooxazolyl, Pyridyl, CONH(C₁-C₂-alkyl), CON(C₁-C₂-alkyl)₂, F oder Cl bedeuten und worin
R₅a bis R₉a Wasserstoff, C₁-C₄-Alkyl, C₁-C₆-Alkoxy, O-Phenyl, O-Benzyl, F, Cl, NH(C₁-C₂-alkyl) oder N(C₁-C₂-alkyl)₂ bedeuten.

Von ganz besonderem Interesse ist ein Verfahren zur Herstellung von
4-Chlorbiphenyl, 4-Chlor-4'-methylbiphenyl, 4-Chlor-4'-n-pentoxybiphenyl oder 4-Chlor-2-methylbiphenyl.

Bevorzugt sind Katalysatoren der Formel (IV), worin R₁ bis R₈ Wasserstoff, Methyl, Ethyl, (C₅-C₆)-Cycloalkyl, Methoxy, Ethoxy, Fluor, NH(C₁-C₂-alkyl), N(C₁-C₂-alkyl)₂, Phenyl,
und R₉, R₁₀, R₁₁, R₁₂ Phenyl, Tolyl, Xylyl, Mesityl, Fluorphenyl oder (C₁-C₄)-Alkoxyphenyl bedeuten,
und Y für Chlorid, Bromid, Jodid, Fluorid, Acetat, Propionat, Benzoat, Sulfat, Hydrogensulfat, Nitrat, Phosphat, Tetrafluoroborat, Tosylat, Mesylat, Acetylacetonat, Hexafluoracetylacetonat,Trifluoracetat oder Pyrazolyl steht.

Besonders bevorzugt sind Verbindungen der Formel (IV) worin R₁ bis R₈ für H, Methyl oder Phenyl, und R₉ bis R₁₂ für Phenyl, Tolyl, Xylyl, Fluorphenyl oder Methoxyphenyl stehen.

Ganz besonders bevorzugt sind
1,1'-Bis(diphenylphosphino)ferrocenpalladium(II)-chlorid (= Pd (dppf)Cl₂),
1,1'-Bis(diphenylphosphino)ferrocenpalladium(II)-chlorid•Dichlormethan und
1,1'-Bis(diphenylphosphino)ferrocenpalladium(II)-bromid.

Der Katalysator wird, bezogen auf den Halogenaromaten der Formel (II), zweckmäßig in der 10⁻⁶ bis 1-fachen, vorzugsweise in der 10⁻⁵ bis 0,1-fachen, insbesondere in der 0,0001 bis 0,01-fachen, molaren Menge eingesetzt. Der Katalysator wird vorzugsweise in homogener Phase eingesetzt. Als Lösungsmittel finden generell inerte organische Lösungsmittel Verwendung. Bevorzugt werden aromatische Lösungsmittel, wie Alkyl-, Dialkyl- oder Trialkylbenzole, oder polar aprotische Lösungsmittel, z.B. Ether, wie Tetrahydrofuran, tert. Butylmethylether, Diethylether, Diethylenglykoldimethylether oder Dimethoxyethan eingesetzt.

Die eingesetzten Palladiumkatalysatoren können vor der erfindungsgemäßen Umsetzung synthetisiert werden, sie können jedoch auch in situ ohne Verlust an katalytischer Aktivität erzeugt werden. Die Synthese des Katalysators erfolgt z.B. analog zu A. W. Rudie, D. W. Lichtenberg, M. L. Katcher, A. Davison, Inorg. Chem. 17, 1978, 2859. Es können auch Mischungen dieser Katalysatoren eingesetzt werden.

Das erfindungsgemäße Verfahren wird im allgemeinen bei Temperaturen von 20 bis 200°C durchgeführt. Bevorzugt sind Temperaturen von 60 bis 180°C, insbesondere 60 bis 100°C.

Von besonderem Vorteil ist die Dosierung der Grignardkomponente, gelöst in einem inerten Lösungsmittel, zum Halogenaromaten und dem Katalysator in einem für alle Reaktionspartner inerten Lösungsmittel. Als inerte Lösungsmittel kommen vorzugsweise die vorstehend für den Katalysator genannten in Betracht.

Die eingesetzten Gridnardverbindungen werden vorteilhaft als 15 bis 40 gew.-%ige Lösungen, z.B. in Tetrahydrofuran eingesetzt. Von besonderem Vorteil sind 20 bis 35 gew.-%ige Lösungen in Tetrahydrofuran.
Die molaren Mengenverhältnisse zwischen den Halogenaromaten der Formel (II) und den Grignardverbindungen der Formel (III) betragen zweckmäßig 1:1 bis 1:1,3, vorzugsweise 1:1,001 bis 1:1,01.

Das erfindungsgemäße Verfahren liefert die gewünschten Produkte in sehr guten Ausbeuten bei sehr guten Umsätzen und Selektivitäten. Nebenprodukte während der Reaktion, gebildet durch Dimerisierung der Grignardkomponente oder Dimerisierung der Halogenaromaten oder durch Folgereaktionen der 4-Chlorbiphenyle mit überschüssigem Grignardreagenz, werden nur in untergeordnetem Maßstab (< 1 %) beobachtet.

Die als Ausgangsstoffe benötigten Verbindungen sind bekannt und können nach an sich bekannten Methoden hergestellt werden.

### Beispiel 1 (4-Chlorbiphenyl)

Zu einer siedenden Lösung von 6 mol 1,4-Bromchlorbenzol und 100 mg Pd(dppf)Cl₂*CH₂Cl₂ in 600 g Tetrahydrofuran (THF) werden innerhalb von 4 Stunden 6 mol einer 26 gew.-%igen Phenylmagnesiumchlorid-THF-Lösung zugetropft. Nach Beendigung der Zugabe wird die Reaktionsmischung weitere 5 Stunden am Rückfluß gekocht. Nach Hydrolyse mit 3600 g 8 gew.-%iger Schwefelsäure, Phasentrennung und anschließender Abdestillation des Lösungsmittels erhält man rohes 4-Chlorbiphenyl, das durch fraktionierte Destillation im Vakuum aufgereinigt wird. Ausbeute 1080 g (96 %), Reinheit des Produktes ≥ 99,7 % (a/a GC).

### Beispiel 2 (4,4'-Dichlorbiphenyl)

Zu einer siedenden Lösung von 0,5 mol 1,4-Brom-chlorbenzol und 0,25 g Pd(dppf)Cl₂*CH₂Cl₂ in 100 ml THF werden innerhalb von 30 Minuten 0,5 mol einer 25 gew.-%igen 4-Chlorphenylmagnesiumchlorid-THF-Lösung zugetropft. Nach vierstündigem Nachrühren unter Rückfluß wird mit verdünnter Schwefelsäure hydrolysiert (Umsatz 99 %, Selektivität 98 %). Die organische Phase wird destillativ vom Lösungsmittel befreit. Das so erhaltene rohe 4,4'-Dichlorbiphenyl wird aus Ethanol umkristallisiert.

### Beispiel 3 (4-Chlor-4'-methylbiphenyl)

Zu einer siedenden Lösung von 0,5 mol 1,4-Brom-chlorbenzol und 0,0025 g Pd(dppf)Cl₂*CH₂Cl₂ in 100 ml THF werden innerhalb von 30 Minuten 0,5 mol einer 25 gew.-%igen 4-Methylphenylmagnesiumchlorid-THF-Lösung zugetropft. Nach vierstündigem Nachrühren unter Rückfluß wird mit verdünnter Schwefelsäure hydrolysiert (Umsatz 99 %, Selektivität 97 %). Die organische Phase wird destillativ vom Lösungsmittel befreit. Das so erhaltene rohe 4-Chlor-4'-methylbiphenyl wird aus Ethanol umkristallisiert.

### Beispiel 4 (4-Chlor-4'-n-pentoxybiphenyl)

Zu einer siedenden Lösung von 0,5 mol 1,4-Brom-chlorbenzol und 0,0025 g Pd(dppf)Cl₂*CH₂Cl₂ in 100 ml THF werden innerhalb von 30 Minuten 0,5 mol einer 25 gew.-%igen 4-n-Pentoxyphenylmagnesiumchlorid-THF-Lösung zugetropft. Nach vierstündigem Nachrühren unter Rückfluß wird mit verdünnter Schwefelsäure hydrolysiert (Umsatz 99 %, Selektivität 97 %). Die organische Phase wird destillativ vom Lösungsmittel befreit. Das so erhaltene rohe 4-Chlor-4'-pentoxybiphenyl wird aus Ethanol umkristallisiert.

### Beispiel 5 (4-Chlor-3'-bis(trimethylsilyl)amino-biphenyl)

Zu einer siedenden Lösung von 0,5 mol 1,4-Brom-chlorbenzol und 0,0025 g Pd(dppf)Cl₂*CH₂Cl₂ in 100 ml THF werden innerhalb von 30 Minuten 0,5 mol einer 25 gew.-%igen 3-Bis(trimethylsilyl)amino-phenylmagnesiumchlorid-THF-Lösung zugetropft. Nach vierstündigem Nachrühren unter Rückfluß wird mit verdünnter Schwefelsäure hydrolysiert (Umsatz 99 %, Selektivität 97 %). Die organische Phase wird destillativ vom Lösungsmittel befreit. Das so erhaltene rohe 4-Chlor-3'bis(trimethylsilyl)amino-biphenyl wird aus Isopropanol umkristallisiert.

### Beispiel 6 Herstellung von 4-Chlor-2-methylbiphenyl

Zu einer siedenden Lösung von 1,0 mol 2-Brom-5-chlortoluol und 0,2 g Pd(dppf)Cl2*CH2Cl2 in 200 mL THF werden innerhalb von 60 Minuten 1,03 mol einer Phenylmagnesiumchlorid-Lösung (26 Gew.-%in THF) zugetropft. Nach achtstündigem Nachrühren unter Rückfluß (Umsatz > 97 %) wird mit Wasser hydrolysiert. Nach destillativer Aufreinigung erhält man 4-Chlor-2-methylbiphenyl als farblose Flüssigkeit in einer Ausbeute von 89 %.

## Patentansprüche

1. Verfahren zur Herstellung von 4-Chlorbiphenylen der Formel (I) 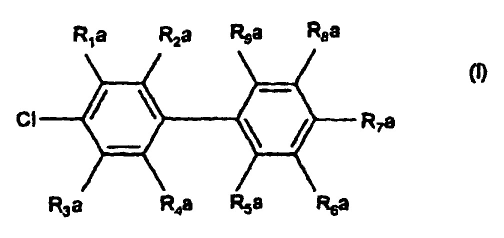 worin
R₁a bis R₄a gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, C₁-C₁₂-Acyloxy, O-Phenyl, O-Benzyl, Aryl, Heteroaryl, F, Cl, NO₂, CN, SO₂R, SOR, NH(C₁-C₆-alkyl), N(C₁-C₆alkyl)₂, C=N(C₁-C₆-alkyl), CX₃, COO-(C₁-C₁₂-alkyl), CO-(C₁-C₁₂-alkyl), CO-Phenyl, COO-Phenyl, CON(C₁-C₈-alkyl)₂, CONH(C₁-C₈-alkyl), CHCHCOO-(C₁-C₁₂-alkyl), N(Si(C₁-C₄-alkyl)₃), PO(Phenyl)₂, PO-(C₁-C₈-alkyl)₂ oder PO₃-(C₁-C₈-alkyl)₂ haben, wobei R Aryl, F oder CₙF₂ₙ₊₁ mit n = 1 bis 12, ist, und wobei X F, Cl oder Br ist; und
R₅a bis R₉a gleich oder verschieden sind und die Bedeutung Wasserstoff, C₁-C₁₂-Alkyl, C₂-C₁₂-Alkenyl, C₂-C₁₂-Alkinyl, C₁-C₁₂-Alkoxy, O-Phenyl, O-Benzyl, Aryl, Heteroaryl, F, Cl, NO₂, CN, SO₂R, SOR, wobei R Aryl ist, haben,
**dadurch gekennzeichnet, daß** ein Halogenaromat der Formel (II) 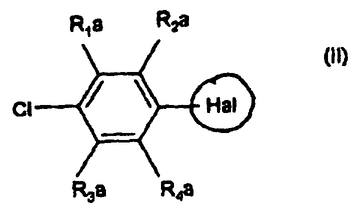 mit einer Arylgrignardverbindung der Formel (III) 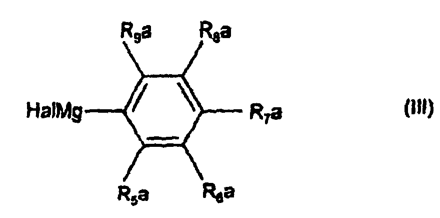 worin Hal für Brom oder Jod steht, in Gegenwart eines Palladiumkatalysators der Formel (IV) 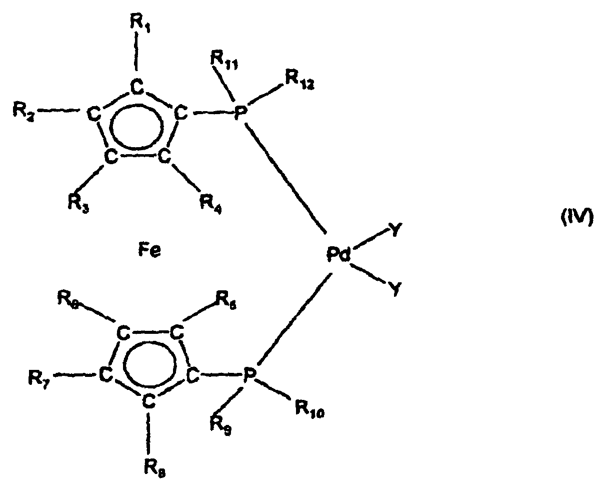 worin
R₁ bis R₈ gleich oder verschieden sind und Wasserstoff, (C₁-C₄)-Alkyl, (C₅-C₈)-Cycloalkyl, (C₁-C₄)-Alkoxy, Fluor, NH₂, NH-(C₁-C₄-Alkyl), N(C₁-C₄-alkyl)₂, CO₂-Alkyl-(C₁-C₄) oder Phenyl bedeuten, oder R₁ und R₂, oder R₂ und R₃, oder R₃ und R₄;
und/oder R₅ und R₆, oder R₆ und R₇, oder R₇ und R₈ zusammen einen aliphatischen oder aromatischen Ring bilden,
R₉ bis R₁₂ gleich oder verschieden sind und C₁-C₈-Alkyl, C₃-C₁₂-Cycloalkyl oder Aryl, das durch 1 bis 3 Substituenten aus der Gruppe C₁-C₄-Alkyl, C₁-C₄-Alkoxy und Halogen substituiert sein kann, bedeuten, und
Y ein Anion einer organischen oder anorganischen Säure ist, umgesetzt wird.

2. Verfahren nach Anspruch 1, **dadurch gekennzeichnet, daß**
R₁a bis R₄a Wasserstoff, C₁-C₈-Alkyl, C₁-C₈-Alkoxy, F, Cl, CN, ein 5- oder 6-gliedriges Heteroaryl mit 1 bis 3 Heteroatomen aus der Gruppe O, S und N, COO-(C₁-C₈-alkyl), CONH(C₁-C₄-alkyl) oder CON(C₁-C₄-alkyl)₂ bedeuten und worin
R₅a bis R₉a Wasserstoff, C₁-C₆-Alkyl, C₂-C₆-Alkenyl, C₁-C₆-Alkoxy, O-Phenyl, O-Benzyl, F, Cl, NH(C₁-C₄-alkyl) oder N(C₁-C₄-alkyl)₂ bedeuten.

3. Verfahren nach Anspruch 1 oder 2, **dadurch gekennzeichnet, daß**
R₁a bis R₄a Wasserstoff, C₁-C₄-Alkyl, C₁-C₄-Alkoxy, CN, COO(C₁-C₄-alkyl), Phenyl, Thiophenyl, Furanyl, Imidazolyl, Oxadiazolyl, Thiadiazolyl, Oxazolyl, 4,5-Dihydrooxazolyl, Pyridyl, CONH(C₁-C₂-alkyl), CON(C₁-C₂-alkyl)₂, F oder Cl bedeuten und worin
R₅a bis R₉a Wasserstoff, C₁-C₄-Alkyl, C₁-C₆-Alkoxy, O-Phenyl, O-Benzyl, F, Cl, NH(C₁-C₂-alkyl) oder N(C₁-C₂-alkyl)₂ bedeuten.

4. Verfahren nach mindestens einem der Ansprüche 1 bis 3, **dadurch gekennzeichnet, daß** die Verbindung der Formel (I) 4-Chlorbiphenyl, 4-Chlor-4'methylbiphenyl, 4-Chlor-2-methylbiphenyl oder 4-Chlor-4'-n-pentoxybiphenyl ist.

5. Verfahren nach mindestens einem der Ansprüche 1 bis 4, **dadurch gekennzeichnet, daß** in der Verbindung der Formel (IV)
R₁ bis R₈ Wasserstoff, Methyl, Ethyl, (C₅-C₆)-Cycloalkyl, Methoxy, Ethoxy, Fluor, NH(C₁-C₂-alkyl), N(C₁-C₂-alkyl)₂, Phenyl,
und R₉, R₁₀, R₁₁, R₁₂ Phenyl, Tolyl, Xylyl, Mesityl, Fluorphenyl oder (C₁-C₄)-Alkoxyphenyl bedeuten,
und Y für Chlorid, Bromid, Jodid, Fluorid, Acetat, Propionat, Benzoat, Sulfat, Hydrogensulfat, Nitrat, Phosphat, Tetrafluoroborat, Tosylat, Mesylat, Acetylacetonat Hexafluoracetylacetonat, Trifluoracetat oder Pyrazolyl steht.

6. Verfahren nach mindestens einem der Ansprüche 1 bis 5, **dadurch gekennzeichnet, daß** in der Verbindung der Formel (IV)
R₁ bis R₈ für H, CH₃ oder Phenyl, und R₉ bis R₁₂ für Phenyl, Tolyl, Xylyl, Fluorphenyl oder Methoxyphenyl stehen.

7. Verfahren nach mindestens einem der Ansprüche 1 bis 6, **dadurch gekennzeichnet, daß** der Katalysator der Formel (IV)
1,1'-Bis(diphenylphosphino)ferrocenpalladium(II)-chlorid,
1,1'-Bis(diphenylphosphino)ferrocenpalladium(II)-chlorid•Dichlormethan oder
1,1'-Bis(diphenylphosphino)ferrocenpalladium(II)-bromid ist.

8. Verfahren nach mindestens einem der Ansprüche 1 bis 7, **dadurch gekennzeichnet, daß** der Katalysator der Formel (IV) in einer 10⁻⁶ bis 1-fachen, vorzugsweise 10⁻⁵ bis 0,1-fachen, molaren Menge, bezogen auf den Halogenaromaten der Formel (II), eingesetzt wird.

## Claims

1. A process for the preparation of 4-chlorobiphenyls of the formula (I) 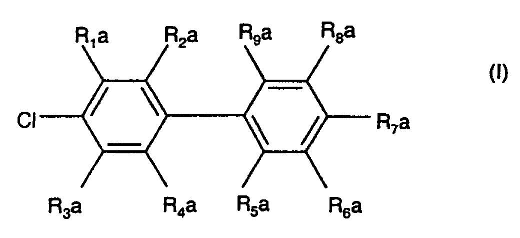 in which
R₁a to R₄a are identical or different and have the meaning hydrogen, C₁-C₁₂-alkyl, C₂-C₁₂-alkenyl, C₂-C₁₂-alkynyl, C₁-C₁₂-alkoxy, C₁-C₁₂-acyloxy, O-phenyl, O-benzyl, aryl, heteroaryl, F, Cl, NO₂, CN, SO₂R, SOR, NH(C₁-C₆-alkyl), N(C₁-C₆-alkyl)₂, C=N(C₁-C₆-alkyl), CX3, COO-(C₁-C₁₂-alkyl), CO-(C₁-C₁₂-alkyl), CO-phenyl, COO-phenyl, CON(C₁-C₈-alkyl)₂, CONH(C₁-C₈-alkyl), CHCHCOO-(C₁-C₁₂-alkyl), N(Si(C₁-C₄-alkyl)₃), PO(phenyl)₂, PO-(C₁-C₈-alkyl)₂ or PO₃-(C₁-C₈-alkyl)₂, where R is aryl, F or CnF₂n+1 where n = 1 to 12, and where X is F, Cl or Br; and
R₅a to R₉a are identical or different and have the meaning hydrogen, C₁-C₁₂-alkyl, C₂-C₁₂-alkenyl, C₂-C₁₂-alkynyl, C₁-C₁₂-alkoxy, O-phenyl, O-benzyl, aryl, heteroaryl, F, Cl, NO₂, CN, SO₂R, SOR, where R is aryl,
which comprises reacting a haloaromatic of the formula (II) 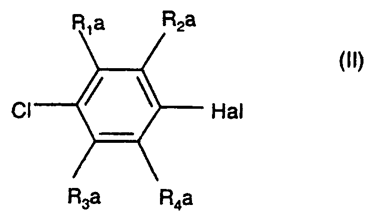 with an aryl Grignard compound of the formula (III) 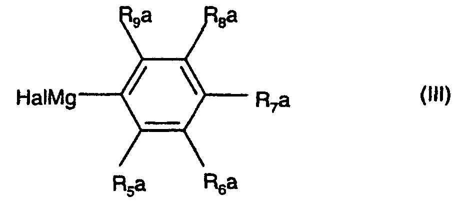 in which Hal is bromine or iodine, in the presence of a palladium catalyst of the formula (IV) 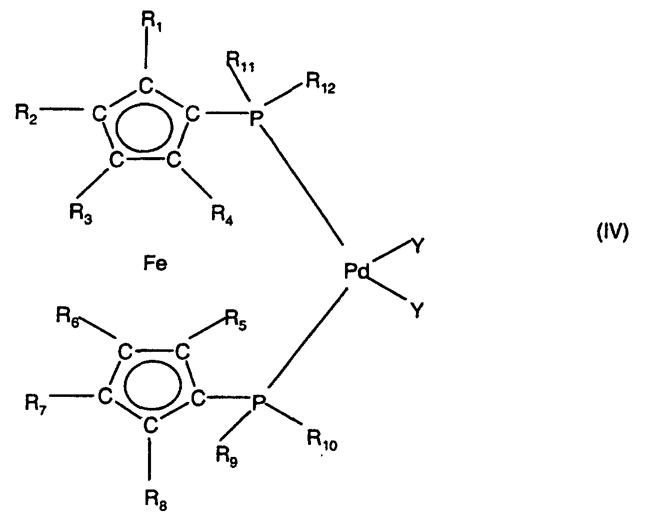 in which
R₁ to R₈ are identical or different and are hydrogen, (C₁-C₄)-alkyl, (C₅-C₈)-cycloalkyl, (C₁-C₄)-alkoxy, fluorine, NH2, NH-(C₁-C₄-alkyl), N(C₁-C₄-alkyl)₂, CO₂-alkyl-(C₁-C₄) or phenyl, or R₁ and R₂, or R₂ and R₃, or R₃ and R₄; and/or R₅ and R₆, or R₆ and R₇, or R₇ and R₈ together form an aliphatic or aromatic ring,
R₉ to R₁₂ are identical or different and are C₁-C₈-alkyl, C₃-C₁₂-cycloalkyl or aryl which can be substituted by 1 to 3 substituents from the group consisting of C₁-C₄-alkyl, C₁-C₄-alkoxy and halogen, and
Y is an anion of an organic or inorganic acid.

2. The process as claimed in claim 1, wherein
R₁a to R₄a are hydrogen, C₁-C₈-alkyl, C₁-C₈-alkoxy, F, Cl, CN, a 5- or 6-membered heteroaryl having 1 to 3 heteroatoms from the group consisting of O, S and N, COO-(C₁-C₈-alkyl), CONH(C₁-C₄-alkyl) or CON(C₁-C₄-alkyl)₂ and in which
R₅a to R₉a are hydrogen, C₁-C₆-alkyl, C₂-C₆-alkenyl, C₁-C₆-alkoxy, O-phenyl, O-benzyl, F, Cl, NH(C₁-C₄-alkyl) or N(C₁-C₄-alkyl)₂.

3. The process as claimed in claim 1 or 2, wherein
R₁a to R₄a are hydrogen, C₁-C₄-alkyl, C₁-C₄-alkoxy, CN, COO(C₁-C₄-alkyl), phenyl, thiophenyl, furanyl, imidazolyl, oxadiazolyl, thiadiazolyl, oxazolyl, 4,5-dihydrooxazolyl, pyridyl, CONH(C₁-C₂-alkyl), CON(C₁-C₂-alkyl)₂, F or Cl and in which
R₅a to R₉a are hydrogen, C₁-C₄-alkyl, C₁-C₆-alkoxy, O-phenyl, O-benzyl, F, Cl, NH(C₁-C₂-alkyl) or N(C₁-C₂-alkyl)₂.

4. The process as claimed in at least one of claims 1 to 3, wherein the compound of the formula (I) is 4-chlorobiphenyl, 4-chloro-4'-methylbiphenyl, 4-chloro-2-methylbiphenyl or 4-chloro-4'-n-pentoxybiphenyl.

5. The process as claimed in at least one of claims 1 to 4, wherein, in the compound of the formula (IV),
R₁ to R₈ are hydrogen, methyl, ethyl, (C₅-C₆)-cycloalkyl, methoxy, ethoxy, fluorine, NH(C₁-C₂-alkyl), N(C₁-C₂-alkyl)₂, phenyl,
and R₉, R₁₀, R₁₁, R₁₂ are phenyl, tolyl, xylyl, mesityl, fluorophenyl or (C₁-C₄)-alkoxyphenyl,
and Y is chloride, bromide, iodide, fluoride, acetate, propionate, benzoate, sulfate, hydrogensulfate, nitrate, phosphate, tetrafluoroborate, tosylate, mesylate, acetylacetonate, hexafluoroacetylacetonate, trifluoracetate or pyrazolyl.

6. The process as claimed in at least one of claims 1 to 5, wherein, in the compound of the formula (IV),
R₁ to R₈ are H, CH3 or phenyl, and R₉ to R₁₂ are phenyl, tolyl, xylyl, fluorophenyl or methoxyphenyl.

7. The process as claimed in at least one of claims 1 to 6, wherein the catalyst of the formula (IV) is
1,1'-bis(diphenylphosphino)ferrocenepalladium(II) chloride,
1,1 '-bis(diphenylphosphino)ferrocenepalladium(II) chloride•dichloromethane or
1,1'-bis(diphenylphosphino)ferrocenepalladium(II) bromide.

8. The process as claimed in at least one of claims 1 to 7, wherein the catalyst of the formula (IV) is employed in a 10-6 to 1-fold, preferably 10-5 to 0.1-fold, molar amount, relative to the haloaromatics of the formula (II).

## Revendications

1. Procédé pour la préparation de 4-chloro-biphényles de formule (I) 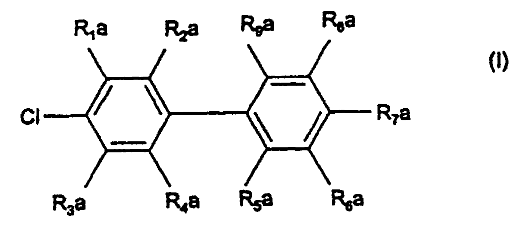 dans laquelle
R₁a à R₄a sont identiques ou différents et représentent un hydrogène, un groupe alkyle en C₁ à C₁₂, alcényle en C₂ à C₁₂, alcynyle en C₂ à C₁₂, alcoxy en C₁ à C₁₂, acyloxy en C₁ à C₁₂, O-phényle, O-benzyle, aryle, hétéroaryle, F, Cl, NO₂, CN, SO₂R, SOR, NH(alkyle en C₁ à C₆), N(alkyle en C₁ à C₆)₂, C=N(alkyle en C₁ à C₆), CX₃, COO-(alkyle en C₁ à C₁₂), CO-(alkyle en C₁ à C₁₂), CO-phényle, COO-phényle, CON(alkyle en C₁ à C₈)₂, CONH(alkyle en C₁ à C₈), CHCHCOO-(alkyle en C₁ à C₁₂), N(Si(alkyle en C₁ à C₄)₃), PO(phényle)₂, PO-(alkyle en C₁ à C₈)₂ ou PO₃-(alkyle en C₁ à C₈)₂, dans lesquels R représente un aryle, F ou CₙF₂ₙ₊₁ avec n = 1 à 12, et dans lesquels X est F, Cl ou Br ; et
R₅a à R₉a sont identiques ou différents et représentent un hydrogène, un groupe alkyle en C₁ à C₁₂, alcényle en C₂ à C₁₂, alcynyle en C₂ à C₁₂, alcoxy en C₁ à C₁₂, O-phényle, O-benzyle, aryle, hétéroaryle, F, Cl, NO₂, CN, SO₂R, SOR, dans lesquels R est un aryle, **caractérisé en ce qu'**on met à réagir un composé halogénoaromatique de formule (II) 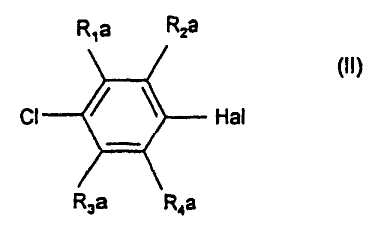 avec un composé de Grignard aryle de formule (III) 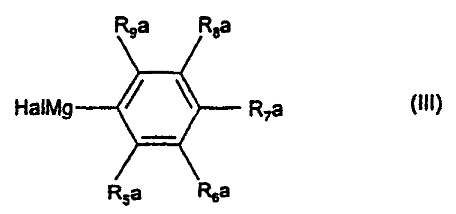 dans laquelle Hal représente un brome ou un iode, en présence d'un catalyseur de palladium de formule (IV) 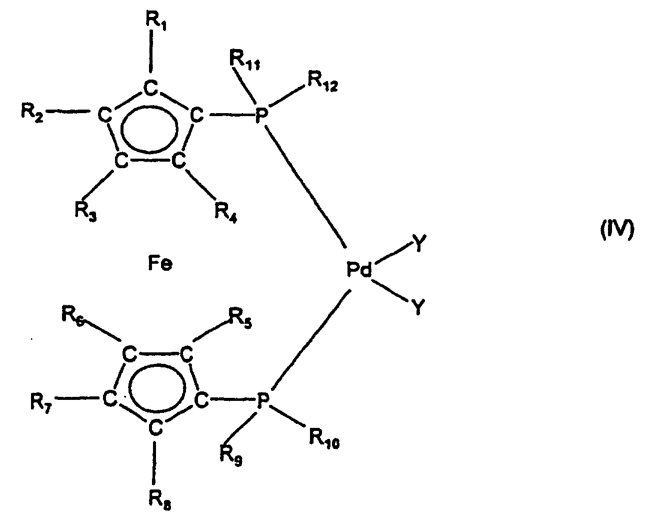 dans laquelle
R₁ à R₈ sont identiques ou différents et représentent un hydrogène, un groupe alkyle en C₁ à C₄, cycloalkyle en C₅ à C₈, alcoxy en C₁ à C₄, fluor, NH₂, NH-(alkyle en C₁ à C₄), N(alkyle en C₁ à C₄)₂, CO₂-alkyle en C₁ à C₄ ou phényle, ou R₁ et R₂, ou R₂ et R₃, ou R₃ et R₄ ; et/ou R₅ et R₆, ou R₆ et R₇, ou R₇ et R₈ forment ensemble un cycle aliphatique ou aromatique,
R₉ à R₁₂ sont identiques ou différents et représentent un groupe alkyle en C₁ à C₈, cycloalkyle en C₃ à C₁₂ ou aryle, qui peut être substitué par 1 à 3 substituants choisis parmi un groupe alkyle en C₁ à C₄, alcoxy en C₁ à C₄ et un halogène, et
Y est un anion d'un acide organique ou inorganique.

2. Procédé selon la revendication 1, **caractérisé en ce que** R₁a à R₄a représentent un hydrogène, un groupe alkyle en C₁ à C₈, alcoxy en C₁ à C₈, F, Cl, CN, un hétéroaryle à 5 ou 6 chaînons ayant 1 à 3 hétéroatomes choisis parmi O, S et N, COO-(alkyle en C₁ à C₈), CONH(alkyle en C₁ à C₄) ou CON(alkyle en C₁ à C₄)₂ et dans laquelle R₅a à R₉a représentent un hydrogène, un groupe alkyle en C₁ à C₆, alcényle en C₂ à C₆, alcoxy en C₁ à C₆, O-phényle, O-benzyle, F, Cl, NH(alkyle en C₁ à C₄) ou N(alkyle en C₁ à C₄)₂.

3. Procédé selon la revendication 1 ou 2, **caractérisé en ce que** R₁a à R₄a représentent un hydrogène, un groupe alkyle en C₁ à C₄, alcoxy en C₁ à C₄, CN, COO(alkyle en C₁ à C₄), phényle, thiophényle, furanyle, imidazolyle, oxadiazolyle, thiadiazolyle, oxazolyle, 4,5-dihydro-oxazolyle, pyridyle, CONH-(alkyle en C₁ à C₂), CON (alkyle en C₁ à C₂)₂, F ou Cl et dans lequel
R₅a à R₉a représentent un hydrogène, un groupe alkyle en C₁ à C₄, alcoxy en C₁ à C₆, O-phényle, O-benzyle, F, Cl, NH(alkyle en C₁ à C₂) ou N(alkyle en C₁ à C₂)₂.

4. Procédé selon au moins une des revendications 1 à 3, **caractérisé en ce que** le composé de formule (I) est le 4-chlorobiphényle, le 4-chloro-4'-méthyl-biphényle, 4-chloro-2-méthylbiphényle ou 4-chloro-4'-n-pentoxybiphényle.

5. Procédé selon au moins une des revendications 1 à 4, **caractérisé en ce que** dans le composé de formule (IV)
R₁ à R₈ représentent un hydrogène, un groupe méthyle, éthyle, cycloalkyle en C₅ à C₆, méthoxy, éthoxy, fluor, NH(alkyle en C₁ à C₂), N(alkyle en C₁ à C₂)₂, phényle,
et R₉, R₁₀, R₁₁, R₁₂ représentent un groupe phényle, tolyle, xylyle, mésityle, fluorophényle ou alcoxyphényle en C₁ à C₄,
et Y représente un chlorure, bromure, iodure, fluorure, acétate, propionate, benzoate, sulfate, hydrogénosulfate, nitrate, phosphate, tétrafluoroborate, tosylate, mésylate, acétylacétonate, hexafluoro-acétylacétonate, trifluoro-acétate ou pyrazolyle.

6. Procédé selon au moins une des revendications 1 à 5, **caractérisé en ce que** dans le composé de formule (IV)
R₁ à R₈ représentent H, CH₃ ou un groupe phényle, et R₉ à R₁₂ représentent un groupe phényle, tolyle, xylyle, fluorophényle et méthoxyphényle.

7. Procédé selon au moins une des revendications 1 à 6, **caractérisé en ce que** le catalyseur de formule (IV) est
le chlorure de 1,1'-bis(diphénylphosphino)ferrocène-palladium(II),
le chlorure de 1,1'-bis(diphénylphosphino)ferrocène-palladium(II)•dichlorométhane ou le bromure de 1,1'-bis(diphénylphosphinoferrocènepalladium(II).

8. Procédé selon au moins une des revendications 1 à 7, **caractérisé en ce que** le catalyseur de formule (IV) est mis en oeuvre en une quantité de 10⁻⁶ à 1 fois, de préférence 10⁻⁵ à 0,1 fois, la quantité molaire, par rapport au composé halogénoaromatique de formule (II).
